# EUROPÄISCHE PATENTSCHRIFT

(11) **EP 2 271 219 B1**
(45) Veröffentlichungstag und Bekanntmachung des Hinweises auf die Patenterteilung: **23.11.2011**
(21) Anmeldenummer: 09778847.5
(22) Anmeldetag: 06.10.2009
(51) Int. Cl.: A01N 43/90, A01P 3/00

(54) **VERWENDUNG VON DITHIIN-TETRACARBOXIMIDEN ZUM BEKÄMPFEN PHYTOPATHOGENER PILZE**
USE OF DITHIIN TETRACARBOXIMIDES FOR TREATING PHYTOPATHOGENIC FUNGI
UTILISATION DE DITHIINE-TETRACARBOXIMIDES POUR LUTTER CONTRE LES CHAMPIGNONS PHYTOPATHOGENES

(30) Priorität: 15.10.2008 EP 08166621
(43) Veröffentlichungstag der Anmeldung: 12.01.2011
(60) Teilanmeldung: 11175741.5
(73) Patentinhaber: Bayer CropScience AG, 40789 Monheim (DE)
(72) Erfinder: SEITZ, Thomas, 40764 Langenfeld (DE); WACHENDORFF-NEUMANN, Ulrike, 56566 Neuwied (DE); BENTING, Jürgen, 42799 Leichlingen (DE); DAHMEN, Peter, 41470 Neuss (DE); VOERSTE, Arnd, 50674 Köln (DE); DUNKEL, Ralf, 42799 Leichlingen (DE); HILLEBRAND, Stefan, 41462 Neuss (DE); TIETJEN, Klaus-Günther, 40764 Langenfeld (DE); BRUNET, Stéphane, 01390 Saint André de Corcy (FR)
(86) Internationale Anmeldenummer: PCT/EP2009/007149
(87) Internationale Veröffentlichungsnummer: WO 2010/043319

(56) Entgegenhaltungen:
- US-A- 3 364 229
- ZENTZ ET AL: "Syntheses, in vitro antibacterial and antifungal activities of a series of N-alkyl, 1,4-dithiines" FARMACO, SOCIETA CHIMICA ITALIANA, PAVIA, IT, Bd. 60, Nr. 11-12, 1. November 2005 (2005-11-01), Seiten 944-947, XP005151567 ISSN: 0014-827X in der Anmeldung erwähnt
- DRABER, WILFRIED: "Synthesis of 1,4-dithiins from maleimide derivatives" CHEMISCHE BERICHTE , 100(5), 1559-70 CODEN: CHBEAM; ISSN: 0009-2940, 1967, XP002563348

## Beschreibung

Die vorliegende Erfindung betrifft die Verwendung von neuen und bekannten Dithiin-tetracarboximiden zum Bekämpfen von phytopathogenen Pilzen, sowie Verfahren zum Bekämpfung phytopathogener Pilze im Pflanzenschutz, sowie Pflanzenschutzmittel enthaltend diese Dithiin-tetracarboximide.

Dithiin-tetracarboximide als solche sind bereits bekannt. Ebenso ist bekannt, dass diese Dithiin-tetracarboximide als Anthelminthika gegen innere Parasiten von Tieren, insbesondere Nematoden, verwendet werden können und insektizide Wirkung aufweisen (vgl. US 3,364,229). Außerdem ist bekannt, das bestimmte Dithiin-tetracarboximide antibakterielle Wirkung besitzen und gegen menschliche Mykosen eine gewisse Wirkung aufweisen (vgl. Il Farmaco, 2005, 60, 944-947). Weiterhin ist bekannt, dass Dithiin-tetracarboximide als Pigment in elektrophotographischen Photorezeptoren oder als Farbstoff in Lacken und Polymeren eingesetzt werden können (vgl. JP-A 10-251265, PL-B 143804).

Da sich die ökologischen und ökonomischen Anforderungen an moderne Fungizide laufend erhöhen, beispielsweise was Wirkungsspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Fungizide zu entwickeln, die zumindest in Teilbereichen die genannten Anforderungen besser erfüllen.

Es wurde nun gefunden, dass Dithiin-tetracarboximide der allgemeinen Formel (I) 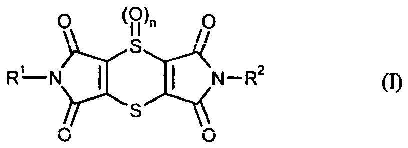 in welcher
- R¹ und R²: gleich oder verschieden sind und für Wasserstoff, für gegebenenfalls einfach oder mehrfach durch Halogen, -OR³, -COR⁴ substituiertes C₁-C₈-Alkyl, gegebenenfalls einfach oder mehrfach durch Halogen, C₁-C₄-Alkyl oder C₁-C₄-Halogenalkyl substituiertes C₃-C₇-Cycloalkyl, jeweils gegebenenfalls einfach oder mehrfach durch Halogen, C₁-C₄-Alkyl, C₁-C₄-Halogenalkyl, -COR⁴ oder Sulfonylamino substituiertes Aryl oder Aryl-(C₁-C₄-alkyl) stehen,
- R³: für Wasserstoff, C₁-C₄-Alkyl, C₁-C₄-Alkylcarbonyl oder für gegebenenfalls einfach oder mehrfach durch Halogen, C₁-C₄-Alkyl oder C₁-C₄-Halogenalkyl substituiertes Aryl steht,
- R⁴: für Hydroxy, C₁-C₄-Alkyl oder C₁-C₄-Alkoxy steht,
- n: für 0 oder 1 steht,
sehr gut zum Bekämpfen phytopathogener Pilze verwendbar sind.

Erfindungsgemäße Dithiin-tetracarboximide der Formel (I) sowie gegebenenfalls deren Salze eignen sich sehr gut zur Bekämpfung pflanzenpathogener Schadpilze. Die vorgenannten erfindungsgemäßen Verbindungen zeigen vor allem eine fungizide Wirksamkeit und lassen sich sowohl im Pflanzenschutz, im Bereich Haushalt und Hygiene als auch im Materialschutz verwenden.

Die erfindungsgemäß verwendbaren Dithiin-tetracarboximide sind durch die Formel (I) allgemein definiert. Bevorzugt verwendbar sind Carboxamide der Formel (I), in welcher die Reste die nachfolgenden Bedeutungen haben.
- R¹ und R²: sind bevorzugt gleich oder verschieden und stehen bevorzugt für Wasserstoff, für gegebenenfalls einfach oder mehrfach durch Fluor, Chlor, Brom, -OR³, -COR⁴ substituiertes C₁-C₆-Alkyl, gegebenenfalls einfach oder mehrfach durch Chlor, Methyl oder Trifluormethyl substituiertes C₃-C₇-Cycloalkyl, für jeweils gegebenenfalls einfach oder mehrfach durch Fluor, Chlor, Brom, Methyl, Trifluormethyl, -COR⁴, Sulfonylamino substituiertes Phenyl oder Phenyl-(C₁-C₄-alkyl)-
- R¹ und R²: sind besonders bevorzugt gleich oder verschieden und stehen besonders bevorzugt für Wasserstoff, für gegebenenfalls einfach oder mehrfach durch Fluor, Chlor, Hydroxy, Methoxy, Ethoxy, Methylcarbonyloxy, Carboxyl substituiertes C₁-C₄-Alkyl, gegebenenfalls einfach oder mehrfach durch Chlor, Methyl oder Trifluormethyl substituiertes C₃-C₇-Cycloalkyl, für jeweils gegebenenfalls einfach bis dreifach durch Fluor, Chlor, Brom, Methyl, Trifluormethyl, -COR⁴, Sulfonylamino substituiertes Phenyl, Benzyl, 1-Phenethyl, 2-Phenethyl oder 2-Methyl-2-phenethyl.
- R¹ und R²: sind ganz besonders bevorzugt gleich oder verschieden und stehen ganz besonders bevorzugt für Wasserstoff, Methyl, Ethyl, n-Propyl, Isopropyl, 2,2-Difluorethyl, 2,2,2-Trifluorethyl, jeweils gegebenenfalls durch Chlor, Methyl oder Trifluormethyl substituiertes Cyclopropyl oder Cyclohexyl.
- R¹ und R²: stehen insbesondere bevorzygt gleichzeitig für Methyl.
- R³: steht bevorzugt für Wasserstoff, Methyl, Ethyl, Methylcarbonyl, Ethylcarbonyl oder für gegebenenfalls einfach oder melufach durch Fluor, Chlor, Methyl, Ethyl, n-Propyl, Isopropyl oder Trifluormethyl substituiertes Phenyl.
- R³: steht besonders bevorzugt für Wasserstoff, Methyl, Methylcarbonyl oder für Phenyl.
- R⁴: steht bevorzugt für Hydroxy, Methyl, Ethyl, Methoxy oder Ethoxy.
- R⁴: steht besonders bevorzugt für Hydroxy oder Methoxy.
- n: steht bevorzugt für 0.
- n: steht auch bevorzugt für 1.
- n: steht besonders bevorzugt für 0.

Im Einzelnen seien folgende Verbindungen genannt:
(1) 2,6-Dimethyl-1H,5H-[1,4]dithiino[2,3-c:5,6-c']dipyrrol-1,3,5,7(2H,6H)-tetron
(2) 2,6-Diethyl-1H,5H-[1,4]dithiino[2,3-c:5,6-c']dipyrrol-1,3,5,7(2H,6H)-tetron
(3) 2,6-Dipropyl-1H,5H-[1,4]dithiino[2,3-c:5,6-c']dipyrrol-1,3,5,7(2H,6H)-tetron
(4) 2,6-Di(propan-2-yl)-1H,5H-[1,4]dithiino[2,3-c:5,6-c']dipyrrol-1,3,5,7(2H,6H)-tetron
(5) 2,6-Dicyclopropyl-1H,5H-[1,4]dithiino[2,3-c:5,6-c']dipyrrol-1,3,5,7(2H,6H)-tetron
(6) 2,6-Bis(2,2,2-trifluorethyl)-1H,5H-[1,4]dithiino[2,3-c:5,6-c']dipyrrol-1,3,5,7(2H,6H)-tetron
(7) 2,6-Bis(trifluormethyl)cyclopropyl]-1H,5H-[1,4]dithiino[2,3:5,6-c']dipyrrol-1,3,5,7(2H,6H)-tetron
(8) 1H,5H-[1,4]Dithiino[2,3-c:5,6-c']dipyrrol-1,3,5,7(2H,6H)-tetron
(9) 2,6-Bis(3,5-dichlorphenyl)-1H,5H-[1,4]dithiino[2,3-c:5,6-c']dipyrrol-1,3,5,7(2H,6H)-tetron
(10) 2,6-Diphenyl-1H,5H-[1,4]dithiino[2,3-c:5,6-c']dipyrrol-1,3,5,7(2H,6H)-tetron
(11) 2,6-Dibenzyl-1H,5H-[1,4]dithiino[2,3-c:5,6-c']dipyrrol-1,3,5,7(2H,6H)-tetron
(12) 2,6-Bis(2-methoxyethyl)-1H,5H-[1,4]dithiino[2,3-c:5,6-c']dipyrrol-1,3,5,7(2H,6H)-tetron
(13) 2,6-Bis(2-hydroxybutyl)-1H,5H-[1,4]dithiino[2,3-c:5,6-c']dipyrrol-1,3,5,7(2H,6H)-tetron
(14) 2,6-Bis(2-hydroxypropyl)-1H,5H-[1,4]dithiino[2,3-c:5,6-c']dipyrrol-1,3,5,7(2H,6H)-tetron
(15) 2,6-Bis(2-phenoxyethyl)-1H,5H-[1,4]dithiino[2,3-c:5,6-c']dipyrrol-1,3,5,7(2H,6H)-tetron
(16) 2,6-Bis(2-ethoxyethyl)-1H,5H-[1,4]dithiino[2,3-c:5,6-c']dipyrrol-1,3,5,7(2H,6H)-tetron
(17) 2,6-Bis(2-phenylpropan-2-yl)-1H,5H-[1,4]dithiino[2,3-c:5,6-c']dipyrrol-1,3,5,7(2H,6H)-tetron
(18) 2,6-Bis(1-phenylethyl)-1H,5H-[1,4]dlthiino[2,3-c:5,6-c']dipyrrol-1,3,5,7(2H,6H)-tetron
(19) 2,6-Bis(2-methoxy-2-methylpropyl)-1H,5H-[1,4]dithiino[2,3-c:5,6-c']dipyrrol-1,3,5,7(2H,6H)-tetron
(20) 2,6-Di-tert-butyl-1H,5H-[1,4]dithiino[2,3-c:5,6-c']dipyrrol-1,3,5,7(2H,6H)-tetron
(21) (1,3,5,7-Tetraoxo-1,3,5,7-tetrahydro-2H,6H-[1,4]dithiino[2,3-c:5,6-c']dipyrrol-2,6-diyl)diethan-2,1-diyl-diacetat
(22) 4,4'-(1,3,5,7-Tetraoxo-1,3,5,7-tetrahydro-2H,6H-[1,4]dithiino[2,3-c:5,6-c']dipyrrol-2,6-diyl)dibenzol-sulfonamid
(23) 2,2'-(1,3,5,7-Tetraoxo-1,3,5,7-tetrahydro-2H,6H-[1,4]dithiino[2,3-c:5,6-c']dipyrrol-2,6-diyl)diessigsäure
(24) 2,2'-(1,3,5,7-Tetraoxo-1,3,5,7-tetrahydro-2H,6H-[1,4]dithiino[2,3-c:5,6-c']dipyrrol-2,6-diyl)dipropansäure
(25) 2,2'-(1,3,5,7-Tetraoxo-1,3,5,7-tetrahydro-2H,6H-[1,4]dithiino[2,3-c:5,6-c']dipyrrol-2,6-diyl)dibutansäure
(26) 2,2'-(1,3,5,7-Tetraoxo-1,3,5,7-tetrahydro-2H,6H-[1,4]dithiino[2,3-c:5,6-c']dipyrrol-2,6-diyl)dihexansäure
(27) 2,2'-(1,3,5,7-Tetraoxo-1,3,5,7-tetrahydro-2H,6H-[1,4]dithiino[2,3-:5,6-c']dipyrrol-2,6-diyl)bis(3,3-di-methylbutansäure)
(28) 3,3'-(1,3,5,7-Tetraoxo-1,3,5,7-tetrahydro-2H,6H-[1,4]dithiino[2,3-c:5,6-c']dipyrrol-2,6-dyl)dibutansäure
(29) 5,5'-(1,3,5,7-Tetraoxo-1,3,5,7-tetrahydro-2H,6H-[1,4]dithiino[2,3-c':5,6-c']dipyrrol-2,6-diyl)dipentansäure
(30) 2,6-Bis[3-(trifluormethyl)cyclohexyl]-1H,5H-[1,4]dithiino[2,3-c:5,6-c']dipyrrol-1,3,5,7(2H,6H)-tetron
(31) 2,6-Bis[3-(trifluormethyl)phenyl]-1H,5H-[1,4]dithiino[2,3-c:5,6-c']dipyrrol-1,3,5,7(2H,6H)-tetron
(32) 2,2'-(1,3,5,7-Tetraoxo-1,3,5,7-tetrahydro-2H,6H-[1,4]dithiino[2,3-c:5,6-c']dipyrrol-2,6-diyl)bis(3-phenylpropansäure
(33) 2,6-Bis(2-hydroxyethyl)-1H,5H-[1,4]dithiino[2,3-c:5,6-c']dipytrol-1,3,5,7(2H,6H)-tetron
(34) 2,6-Bis(2-hydroxy-2-methylpropyl)-1H,5H-[1,4]dithiino[2,3-c:5,6-c']dipyrrol-1,3,5,7(2H,6H)-tetron
(35) (1,3,5,7-Tetraoxo-1,3,5,7-tetrahydro-2H,6H-[1,4]dithiino[2,3-c:5,6-c']dipyrrol-2,6-diyl)dibutan-1,2-diyl-diacetat
(36) (1,3,5,7-Tetraoxo-1,3,5,7-tetrahydro-2H,6H-[1,4]dithiino[2,3-c:5,6-c']dipyrrol-2,6diyl)dipropan-1,2-diyldiacetat
(37) 2,6-Bis(hydroxymethyl)-1H,5H-[1,4]dithiino[2,3-c:5,6-c']dipyrol-1,3,5,7(2H,6H)-tetron
(38) 2,6-Dimethyl-1H,5H-[1,4]dithiino[2,3-c:5,6-c']dipyrrol-1,3,5,7(2H,6H)-tetron-4-oxid
(39) 2-Ethyl-1H,5H-[1,4]dithiino[2,3-c:5,6-c']dipyrrol-1,3,5,7(2H,6H)-tetron
(40) Diethyl-2,2'-(1,3,5,7-tetraoxo-1,3,5,7-tetrahydro-2H,6H-[1,4]dithiino[2,3-c:5,6-c']dipyrrol-2,6-diyl)dihexanoat
(41) 2-[2-(1-Ethoxy-1-oxobutan-2-yl)-1,3,5,7-tetraoxo-2,3,5,7-tetrahydro-1H,6H-[1,4]dithiino[2,3-c:5,6-c']dipyrrol-6-yl]butansäure

Besonders bevorzugt verwendbar sind die Verbindungen (1), (2) und (3).

Auch erfindungsgemäß sind Dithiin-tetracarboximide der Formel (I-a) 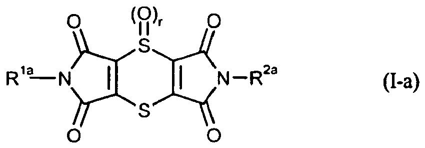 in welcher
- R^{1a} und R^{2a}: gleich oder verschieden sind und für einfach oder mehrfach durch Fluor, -OR^{3a}, -COR^{4a} substituiertes C₁-C₈-Alkyl, gegebenenfalls einfach oder mehrfach durch Halogen, C₁-C₄-Alkyl oder C₁-C₄-Halogenalkyl substituiertes cyclopropyl oder für einfach im Alkylteil durch -COR^{4a} substituiertes Aryl-(C₁-C₄-alkyl) stehen,
- R^{3a}: für C₁-C₄-Alkyl oder für gegebenenfalls einfach oder mehrfach durch Halogen, C₁-C₄-Alkyl oder C₁-C₄-Halogenalkyl substituiertes Aryl steht,
- R⁴': für Hydroxy, C₁-C₄-Alkyl oder C₁-C₄-Alkoxy steht,
- r: für 0 oder 1 steht,
- wobei R^{1a} und R^{2a}: nicht gleichzeitig für Acetoxymethyl oder Methoxymethyl stehen.
- R^{1a} und R^{2a}: sind Bevorzugt gleich oder verschieden und stehen bevorzugt für einfach oder mehrfach durch Fluor, -OR^{3a}, -COR^{4a} substituiertes C₁-C₆-Alkyl, gegebenenfalls einfach oder mehrfach durch Chlor, Methyl oder Trifluormethyl substituiertes, oder für einfach im Alkylteil durch -COR⁴' substituiertes Phenyl-(C₁-C₄-alkyl).
- R^{1a} und R^{2a}: sind besonders bevorzugt gleich oder verschieden und stehen besonders bevorzugt für einfach oder mehrfach durch Fluor, Hydroxy, Methoxy, Ethoxy, Methylcarbonyloxy, Carboxyl substituiertes C₁-C₄-Alkyl, gegebenenfalls einfach oder mehrfach durch Chlor, Methyl oder Trifluormethyl substituiertes oder für einfach im Alkylteil durch -COR^{4a} substituiertes 1-Phenethyl oder 2-Phenethyl.
- R^{1a} und R^{2a}: sind ganz besonders bevorzugt gleich oder verschieden und stehen ganz besonders bevorzugt für 2,2-Difluorethyl, 2,2,2-Trifluorethyl, jeweils gegebenenfalls durch Chlor, Methyl oder Trifluormethyl substituiertes Cyclopropyl.
- R^{3a}: steht bevorzugt für Methyl, Ethyl, oder für gegebenenfalls einfach oder mehrfach durch Fluor, Chlor, Methyl, Ethyl, n-Propyl, Isopropyl oder Trifluormethyl substituiertes Phenyl.
- R^{3a}: steht besonders bevorzugt für Methyl oder für Phenyl.
- R^{4a}: steht bevorzugt für Hydroxy, Methyl, Ethyl, Methoxy oder Ethoxy.
- R^{4a}: steht besonders bevorzugt für Hydroxy oder Methoxy.
- r: steht bevorzugt für 0.
- r: steht auch bevorzugt für 1.
- r: steht besonders bevorzugt für 0.

Je nach Art der oben definierten Substituenten können die Verbindungen der Formel (I) saure oder basische Eigenschaften aufweisen und können mit anorganischen oder organischen Säuren oder mit Basen oder mit Metallionen Salze, gegebenenfalls auch innere Salze oder Addukte bilden.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Tragen die Verbindungen der Formel (I) Hydroxy, Carboxy oder andere, saure Eigenschaften induzierende Gruppen, so können diese Verbindungen mit Basen zu Salzen umgesetzt werden.

Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkylresten, Mono-, Di- und Trialkanolamine von (C₁-C₄)-Alkanolen, Cholin sowie Chlorcholin.

Tragen die Verbindungen der Formel (I) Amino, Alkylamino oder andere, basische Eigenschaften induzierende Gruppen, so können diese Verbindungen mit Säuren zu Salzen umgesetzt werden.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄.

Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl welche ein oder zwei Sulfonsäuregruppen tragen), Alkylphosphon-säuren (Phosphonsäuren mit geradkettigen oder verzweigten Alkylresten mit 1 bis 20 Kohlenstoffatomen), Arylphosphonsäuren oder - diphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc.

Die so erhältlichen Salze weisen ebenfalls fungizide Eigenschaften auf.

Die erfindungsgemäß verwendbaren Dithiin-tetracarboximide der Formel (I) können auf bekannte Weise hergestellt werden (vgl. US 3,364,229, Synthetic Commun. 2006, 36, 3591-3597 und Il Farmaco 2005, 60 944-947).

Beispielsweise wird in einem ersten Verfahren (vgl. Il Farmaco 2005, 60, 944-947) in einer ersten Stufe Bernsteinsäureanhydrid der Formel (II) mit einem Amin der Formel (III) gegebenenfalls in Gegenwart eines Verdünndungsmittels umgesetzt. Anschließend werden dann die so erhaltenen Bemsteinsäuremonoamide der Formel (IV) mit einer Schwefelquelle (z.B. Thionylchlorid) umgesetzt. Je nach Reaktionsbedingungen können die Dithiin-diisoimide der Formel (V) isoliert werden, bevor man sie in die Dithiin-tetracarboximide der Formel (I-b) überführt. Die Herstellung der Dithiin-tetracarboximide der Formel (I) kann durch das folgende Schema veranschaulicht werden (R steht hierin für R¹ oder R²):

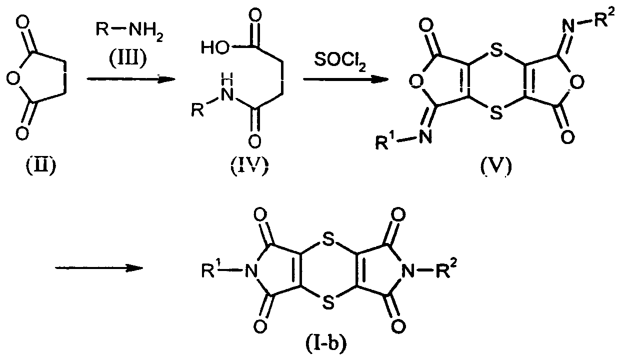

Auch die Dithiin-diisoimide der Formel (V) 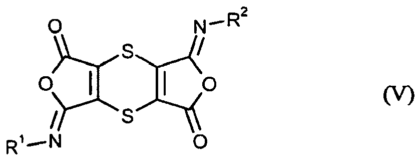 in welcher R¹ und R² die oben angegebenen Bedeutungen haben, eigenen sich zum Bekämpfen phytopathogener Pilze.

R¹ und R² haben dabei die oben angegebenen bevorzugten, besonders bevorzugten, ganz besonders bevorzugten bzw. insbesondere bevorzugten Bedeutungen.

Auch erfindungsgemäß sind Dithiin-diisoimide der Formel (V-a) 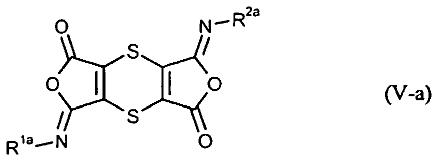 in welcher R^{1a} und R^{2a} die oben angegebenen Bedeutungen haben.

R^{1a} und R^{2a} haben dabei die oben angegebenen bevorzugten, besonders bevorzugten, ganz besonders bevorzugten bzw. insbesondere bevorzugten Bedeutungen.

Beispielsweise wird in einem zweiten Verfahren (vgl. US 3,364,229, Synthetic Commun. 2006, 36, 3591-3597) in einer ersten Stufe Dichlormaleinsäureanhydrid der Formel (VI) mit einem Amin der Formel (III) gegebenenfalls in Gegenwart eines Verdünndungsmittels umgesetzt. Anschließend werden dann die so erhaltenen Maleinsäureimide der Formel (VII) mit einer Schwefelquelle (z.B. Schwefelwasserstoff oder Thioharnstoff) umgesetzt. Die entstandenen Dithiin-tetracarboximide der Formel (I-b) können gegebenenfalls anschließend mit Salpetersäure oxidiert werden. Die Herstellung der Dithiin-tetracarboximide der Formel (I) kann durch das folgende Schema veranschaulicht werden (R steht hierin für R¹ oder R²):

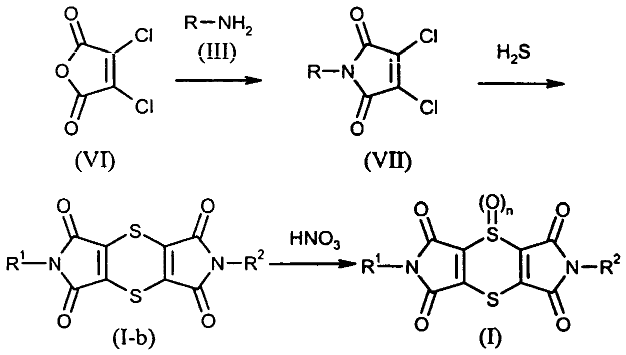

Die vorliegende Erfindung betrifft weiterhin ein Pflanzenschutzmittel zum Bekämpfen unerwünschter Pilze umfassend wenigstens ein Dithiin-tetracarboximid der Formel (I) oder ein Dithiin-diisoimide der Formel (V). Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Außerdem betrifft die Erfindung ein Verfahren zum Bekämpfen unerwünschter Mikroorganismen, dadurch gekennzeichnet, dass man erfindungsgemäß Dithiin-tetracarboximide der Formel (I) oder ein Dithiin-diisoimide der Formel (V) auf die phytopathogenen Pilze und/oder deren Lebensraum ausbringt. Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel.

Als verflüssigte gasförmige Streckmittel oder Trägerstoffe kommen solche Flüssigkeiten infrage, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Dichlormethan, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsnüttel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder A-rylphenole), Salze von Sulphobemsteinsäureestem, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiennittel, Komplexbildner. Im Allgemeinen können die Wirkstoffe mit jedem festen oder flüssigen Additiv, welches für Formulierungszwecke gewöhnlich verwendet wird, kombiniert werden.

Die Formulierungen enthalten im Allgemeinen zwischen 0,05 und 99 Gew.-%, 0,01 und 98 Gew.-%, vorzugsweise zwischen 0,1 und 95 Gew.%, besonders bevorzugt zwischen 0,5 und 90 % Wirkstoff, ganz besonders bevorzugt zwischen 10 und 70 Gewichtsprozent.

Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate. Öl dispergierbare Pulver, Öl mischbare fließfähige Konzentrate, Öl nüschbare Flüssigkeiten, Schäume, Pasten, Pestizid ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgutbehandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schtämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die Erfindung umfasst weiterhin ein Verfahren zur Behandlung von Saatgut.

Die Erfindung betrifft weiterhin Saatgut, welches gemäß einem der im vorherigen Absatz beschriebenen Verfahren behandelt wurde. Die erfindungsgemäßen Saatgüter finden Anwendung in Verfahren zum Schutz von Saatgut vor unerwünschten Pilzen. Bei diesen wird ein mit wenigstens einem erfindungsgemäßen Wirkstoff behandeltes Saatgut verwendet.

Die erfindungsgemäßen Wirkstoffe bzw. Mittel sind auch geeignet für die Behandlung von Saatgut. Ein großer Teil des durch Schadorganismen hervorgenifenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt werden, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von phytopathogenen Pilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit einem erfindungsgemäßen Mittel behandelt wurde.

Die Bekämpfung von phytopathogenen Pilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Wirkstoffe bzw. Mittel die Behandlung des Saatguts mit diesen Wirkstoffen bzw. Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwutschaft, im Gewächshaus, in Forsten oder im Garten- und Weinbau eingesetzt wird. Insbesondere handelt es sich dabei um Saatgut von Getreide (wie Weizen, Gerste, Roggen, Triticale, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Raps, Mohn, Olive, Kokosnuss, Kakao, Zuckerrohr, Tabak, Gemüse (wie Tomate, Gurke, Zwiebeln und Salat), Rasen und Zierpflanzen (siehe auch unten). Besondere Bedeutung kommt der Behandlung des Saatguts von Getreide (wie Weizen, Gerste, Roggen, Triticale und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus or Gliocladium stammen. Bevorzugt stammt dieses heterologe Gen aus Bacillus sp., wobei das Genprodukt eine Wirkung gegen den Maiszünsler (European com borer) und/oder Western Com Rootworm besitzt. Besonders bevorzugt stammt das heterologe Gen aus Bacillus thuringiensis.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geemtet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfmdungsgemäßen Mittel können ummittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfmdungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die erfindungsgemäßen Wirkstoffe bzw. Mittel weisen eine starke fungizide Wirkung auf und können zur Bekämpfung von unerwünschten Pilzen im Pflanzenschutz und im Materialschutz eingesetzt werden.

Die erfindungsgemäßen Dithiin-tetracarboximide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht wird.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes. Sie können vorzugsweise als Pflanzenschutzmittel eingesetzt werden. Sie sind gegen normal sensible und resistente Arten sowie gegen alle oder einzelne Entwicklungsstadien wirksam.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), Ri*besioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und -plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp*. (beispielsweise Sojabohne), *Solanacene sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, dass es ein interessierendes Protein oder Polypeptid exprimiert oder dass es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verfubeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, dass die behandelten Pflanzen, wenn sie im Anschluss daran mit unerwünschten phytopathogenen Pilzen inokuliert wurde, einen beträchtlichen Resistenzgrad gegen diese unerwünschten phytopathogenen Pilze aufweisen. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfingdungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Stressfaktoren resistent, d.h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Stressfaktoren resistent sind. Zu den abiotischen Stressbedingungen können zum Beispiel Dürre, Kälte- und Hitzebedingungen, osmotischer Stress, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Stress- und Nicht-Stress-Bedingungen) beeinflusst werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Stressfaktoren führt. Solche Pflanzen werden typischerweise dadurch erzeugt, dass man eine ingezüchtete pollensterile Eltemlinie (den weiblichen Kreuzungspartner) mit einer anderen ingezüchteten pollenfertilen Eltemlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättem exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.*, die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, dass man ein Gen exprimiert, das für ein Glyphosate-Oxidoreduktase-Enzym kodiert. Glyphosatetolerante Pflanzen können auch dadurch erhalten werden, dass man ein Gen exprimiert, das für ein Glyphosate-acetyltransferase-Enzym kodiert. Glyphosatetolerante Pflanzen können auch dadurch erhalten werden, dass man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmem tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmem kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmem kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht werden sind. Zu bekannten ALS-Hemmem zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylhamstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfasst im vorliegenden Zusammenhang jegliche Pflanze, die mindestens ein Transgen enthält, das eine Kodiersequenz umfasst, die für folgendes kodiert:
1) ein insektizides Kristallprotein aus *Bacillus thuringiensis* oder einen insektiziden Teil davon, wie die insektiziden Kristallproteine, die online bei:
   http://www.lifesci.sussex.ac.uk/Home/Neil_Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfasst, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezemiertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezemiertes Protein aus *Bacillus thuringiensis* oder *Bacillus cereus,* das in Gegenwart eines zweiten sezemierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfasst, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfasst, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Stressfaktoren tolerant. Solche Pflanzen können durch genetische Transformation oder durch Selektion von Pflanzen, die eine Mutation enthalten, die solch eine Stressresistenz verleiht, erhalten werden. Zu besonders nützlichen Pflanzen mit Stresstoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein stresstoleranzfördemdes Transgen enthalten, das die Expression und/oder Aktivität der für PARG kodierenden Gene der Pflanzen oder Pflanzenzellen zu reduzieren vermag;
c. Pflanzen, die ein stresstoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Emteprodukts auf, wie zum Beispiel:
1) Transgene Pflanzen, die eine modifizierte Stärke synthetisieren, die bezüglich ihrer chemischphysikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekomgröße und/oder Stärkekomrnorphologie im Vergleich mit der synthetisierten Stärke in Wildtyppflanzenzellen oder -pflanzen verändert ist, so dass sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Bamwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylhamstofioleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die erfindungsgemäßen Wirkstoffe bzw. Mittel können außerdem im Materialschutz zum Schutz von technischen Materialien gegen Befall und Zerstörung durch unerwünschte Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor pilzlicher Veränderung oder Zerstörung geschützt werden sollen, Klebstoffe, Leime, Papier, Wandpappe und Karton, Textilien, Teppiche, Leder, Holz, Anstrichmittel und Kunststoffartikel, Kühlschnlierstoffe und andere Materialien sein, die von Mikroorganismen befallen oder zersetzt werden können. Im Rahmen der zu schützenden Materialien seien auch Teile von Produktionsanlagen und Gebäuden, z.B. Kühlwasserkreisläufe, Kühl- und Heizsysteme und Belüftungs- und Klimaanlagen, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern. Außerdem können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Das erfindungsgemäße Verfahren zum Bekämpfen von unerwünschten Pilzen kann auch zum Schutz von so genannten Storage Goods venvendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Beispielhaft, aber nicht begrenzend, seien einige Erreger von pilzlichen" Erkrankungen, die erfindungsgemäß behandelt werden können, genannt:

Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator; Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi und Phakopsora meibomiae; Puccinia-Arten, wie beispielsweise Puccinia recondita oder Puccinia triticina; Uromyces-Arten, wie beispielsweise Uromyces appendiculatus;
Erkrankungen, hervorgerufen durch Erreger der Gruppe der Oomyceten wie z.B. Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Altemaria solani; Cercospora-Arten, wie beispielsweise Cercospora beticola; Cladiosporum-Arten, wie beispielsweise Cladiosporium cucumerinum; Cochliobolus-Arten, wie beispielsweise Cochliobolus sativus (Konidienform: Drechslera, Syn: Helminthosporium); Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola und M. fijiensis; Phaeosphaeria-Arten, wie beispielsweise Phaeosphaeria nodorum; Pyrenophora-Arten, wie beispielsweise Pyrenophora teres; Ramuiaria-Arten, wie beispielsweise Ramularia collo-cygni; Rhynchosporium-Arten, wie beispielsweise Rhynchosporium secalis; Septoria-Arten, wie beispielsweise Septoria apii; Typhula-Arten, wie beispielsweise Typhula incarnata; Venturia-Arten, wie beispielsweise Venturia inaequalis;
Wurzel- und Stängetkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Rhizoctonia-Arten, wie beispielsweise Rhizoctonia solani; Tapesia-Arten, wie beispielsweise Tapesia acuformis; Thielaviopsis-Arten, wie beispielsweise Thielaviopsis basicola; Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Septoria-Arten, wie beispielsweise Septoria nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries, T. controversa; Urocystis-Arten, wie beispielsweise Urocystis occulta; Ustilago-Arten, wie beispielsweise Ustilago nuda, U. nuda tritici; Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum und P. purpurogenum; Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B. Fusarium-Arten, wie beispielsweise Fusarium culmorum; Phytophthora Arten, wie beispielsweise Phytophthora cactorum; Pythium-Arten, wie beispielsweise Pythium ultimum; Rhizoctonia-Arten, wie beispielsweise Rhizoctonia solani; Sclerotium-Arten, wie beispielsweise Selerotium rolfsii; Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa; Deformationen von Blättern, Blüten und Früchten, hervorgerufen durch z.B. Taphrina-Arten, wie beispielsweise Taphrina deformans;
Degenerationserkrankungen holziger Pflanzen, hervorgerufen durch z.B. Esca-Arten, wie beispielsweise Phaemoniella clamydospora und Phaeoacremonium aleophilum und Fomitiporia mediterranea;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;

Bevorzugt können die folgenden Krankheiten von Soja-Bohnen bekämpft werden:
Pilzkrankheiten an Blättern, Stängeln, Schoten und Samen verursacht durch z.B. Altemaria leaf spot (Alternaria spec. atrans tenuissima), Anthracnose (Colletotrichum gloeosporoides dernatium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyffosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).
Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Als Organismen, die einen Abbau oder eine Veränderung der technischen Materialien bewirken können, seien Pilze genannt. Vorzugsweise wirken die erfindungsgemäßen Wirkstoffe gegen Pilze, insbesondere Schimmelpilze, Holz verfärbende und Holz zerstörende Pilze (Basidiomyceten). Es seien beispielsweise Pilze der folgenden Gattungen genannt: Alternaria, wie Alternaria tenuis; Aspergillus, wie Aspergillus niger, Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride.

Darüber hinaus weisen die erfindungsgemäßen Wirkstoffe auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Beim Einsatz der erfindungsgemäßen Wirkstoffe als Fungizide können die Aufwandmengen je nach Applikationsart innerhalb cines größeren Bereiches variiert werden. Die Aufwandmenge der erfindungsgemäßen Wirkstoffe beträgt
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2-und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkomalkaloide und Aflatoxine, die beispielsweise von den folgenden Pilzen verursacht werden können: Fusarium spec., wie Fusarium acunimatum, F. avenaceum, F. crookwellense, F. culmorum, F. graminearum (Gibberella zeae), F. equiseti, F. fujikoroi, F. musarun, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

Die aufgeführten Pflanzen können besonders vorteilhaft erfindungsgemäß mit den Verbindungen der allgemeinen Formel (I) oder ein Dithiin-diisoimide der Formel (V) den erfindungsgemäßen Mitteln behandelt werden. Die bei den Wirkstoffen bzw. Mitteln oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mitteln.

### Herstellungsbeispiele

### Herstellung von 2,6-Bis[1-(trifluormethyl)cyclopropyl]-1H,5H-[1,4]dithiino[2,3-c:5,6-c']dipyrrol-1,3,5,7-(2H,6H)-tetron [Verbindung Nr. (7)]

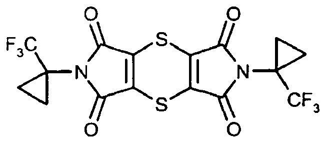

Zu einer Lösung von 0,8 g (3,55 mmol) 4-Oxo-4-{[1-(trifluormethyl)cyclopropyl]amino}butansäure (IV-1) in 2 mL Dioxan wurden unter Eiskühlung (15°C) langsam 7,57 mL (103,75 mmol) Thionylchlorid zugetropft. Man ließ über Nacht auf Raumtemperatur erwärmen und engte die Lösung ein. Der Rückstand wird auf Eis gegossen, mit Essigsäureethylester extrahiert, getrocknet und eingeengt. Nach Chromatographie an Kieselgel (Cyclohexan/Essigsäureethylester 1:1) erhält man 284 mg (34 % der Theorie) der gewünschten Verbindung.

### Herstellung von (1,3,5,7-Tetraoxo-1,3,5,7-tetrahydro-2H,6H-[1,4]dithiino[2,3-c:5,6-c']dipyrrol-2,6-diyl)dipropan-1,2-diyldiacetat [Verbindung Nr. (36)1

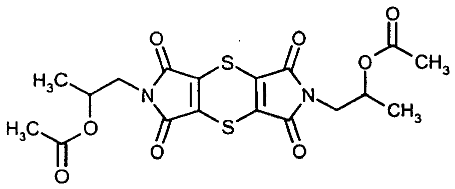

Zu einer Lösung von 1,1 g (3,72 mmol) 1-(3,4-Dichlor-2,5-dioxo-2,5-dihydro-1H-pyrrol-1-yl)propan-2-yl-acetat in 10 mL Ethanol gibt man 0,283 g (3,72 mmol) an Thioharnstoff und lässt 5 Stunden bei 40°C nachrühren. Nach Abkühlung auf Raumtemperatur saugt man grüne Kristalle ab, die mit Wasser/Ethanol nachgewaschen werden. Das Filtrat wird mit Wasser und Essigsäureethylester extrahiert, getrocknet und eingeengt. Die Mutterlauge wird an Kieselgel (Gradient Cyclohexan / Essigsäureethylester 0 % → 100 %) chromatographiert. Man erhält 0,334 g (39,5 % der Theorie) der gewünschten Verbindung.

### Herstellung von 2,6-Dimethyl-1H,5H-[1,4]dithiino[2,3-c:5,6-c']dipyrrol-1,3,5,7(2H,6H)-tetron-4-oxid [Verbindung] Nr. (38)]

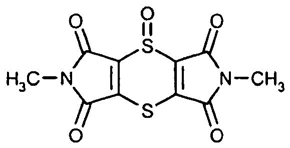

Zu 20 mL eisgekühlter (5°C) rauchender Salpetersäure gibt man unter Rühren 3 g (10,63 mmol) 2,6-Dimethyl-1H,5H-[1,4]dithiino[2,3-c:5,6-c']dipyrrol-1,3,5,7(2H,6H)-tetron [Verbindung Nr. (1)]. Nach vollständiger Lösung rührt man 5 min weiter, gießt anschließend auf Eiswasser und saugt die gelben Kristalle ab. Man erhält 2,56 g (80,8 % der Theorie) von der gewünschten Verbindung.

Analog den vorangehenden Beispielen sowie entsprechend den allgemeinen Beschreibungen der Verfahren können die in der folgenden Tabelle 1 genannten Verbindungen der Formel (I) erhalten werden.

**Tabelle 1**

| | | | | |
|---|---|---|---|---|
| 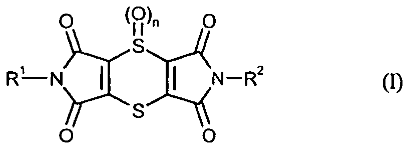 | | | | |

| Nr. | R¹ | R² | n | Physikalische Daten |
|---|---|---|---|---|
| 1 | Me | Me | 0 | Log P = 2,32 |
| 2 | Et | Et | 0 | ¹H-NMR (400 MHz, DMSO-d₆): δ = 1,096; 3,442 ppm |
| 3 | nPr | nPr | 0 | ¹H-NMR (400 MHz, DMSO-d₆): δ = 0,822; 1,566; 3,362 ppm |
| 4 | iPr | iPr | 0 | Log P = 4,19 |
| 5 | cPr | cPr | 0 | ¹H-NMR (400 MHz, DMSO-d₆): δ = 0,50 -0,89 ppm |
| 6 | -CH₂CF₃ | -CH₂CF₃ | 0 | Log P = 3,41 |
| 7 | 1-(Trifluormethyl)-cyclopropyl | 1-(Trifluormethyl)-cyclopropyl | 0 | Log P =4 03 |
| 8 | H | H | 0 | Log P = 1,13 |
| 9 | 3,5-Dichlorphenyl | 3,5-Dichlorphenyl | 0 | Fp. > 300°C |
| 10 | Ph | Ph | 0 | Fp. > 300°C |
| 11 | Bz | Bz | 0 | Log P = 4,60 |
| 12 | 2-Methoxyethyl | 2-Methoxyethyl | 0 | Log P = 2,55 |
| 13 | 2-Hydroxybutyl | 2-Hydroxybutyl | 0 | Log P = 2,27 |
| 14 | 2-Hydroxypropyl | 2-Hydroxypropyl | 0 | Log P = 1,63 |
| 15 | 2-Phenoxyethyl | -Phenoxyethyl | 0 | Log P = 3,86 |
| 16 | 2-Ethoxyethyl | 2-Ethoxyethyl | 0 | Log P = 3,24 |
| 17 | Phenylpropan-2-yl | 2-Phenylpropan-2-yl | 0 | ¹H-NMR (400 MHz, DMSO-d₆): δ = 7,20 -7,35 ppm |
| 18 | 1-Phenylethyl | 1-Phenylethyl | 0 | ¹H-NMR (400 MHz, DMSO-d₆): δ = 5,197; 0 5,215; 5,234; 5,251 ppm |
| 19 | 2-Methoxy-2-methylpropyl | 2-Methoxy-2-methylpropyl | 0 | |
| 20 | tBu | tBu | 0 | |
| 21 | -(CH₂)₂OC(=O)CH₃ | -(CH₂)₂OC(=O)CH₃ | 0 | ¹H-NMR (400 MHz, DMSO-d₆): δ = 1,053; 3,654; 4,110 ppm |
| 22 | 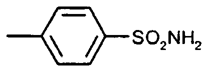 | 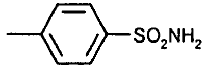 | 0 | ¹H-NMR (400 MHz, DMSO-d₆): δ = 7,492; 7,596; 7,583; 7,946; 7,966 ppm |
| 23 | -CH₂CO₂H | -CH₂CO₂H | 0 | ¹H-NMR (400 MHz, DMSO-d₆): δ = 4,166 ppm |
| 24 | 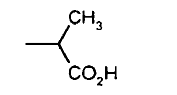 | 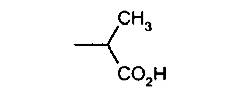 | 0 | Log P = 1,76 |
| 25 | 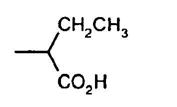 | 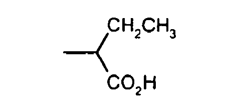 | 0 | |
| 26 | 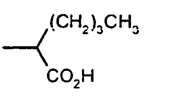 | 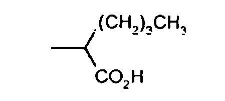 | 0 | |
| 27 | 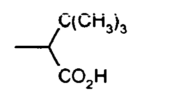 | 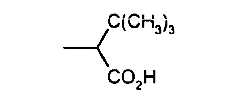 | 0 | ¹H-NMR (400 MHz, DMSO-d₆): δ = 1,620 ppm |
| 28 | 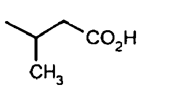 | 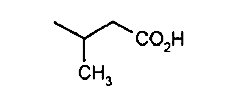 | 0 | Log P = 1,99 |
| 29 | -(CH₂)₄CO₂H | -(CH₂)₄CO₂H | 0 | Log P = 2,02 |
| 30 | 3--(Trifluormethyl)-cyclohexyl | 3-(Trifluomethyl)-cyclohexyl | 0 | ¹³C-NMR (150 MHz, DMSO-d₆): δ = 23,01; 23,71; 27,85; 28,61; 49,19; 126,77; 128,62; 130,56; 164,22 |
| 31 | 3--(Trifluormethyl)-phenyl | 3-(Trifluormethyl)-phenyl | 0 | ppm Log P = 4,91 |
| 32 | 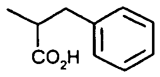 | 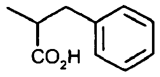 | 0 | Log P = 3,12 |
| 33 | 2-Hydroxyethyl | 2-Hydroxyethyl | 0 | ¹H-NMR (400 MHz, DMSO-d₆): δ = 3,480 ppm |
| 34 | 2-Hydroxy2-methylpropyl | 2-Hydroxy2-methylpropyl | 0 | Log P = 3,65 |
| 35 | 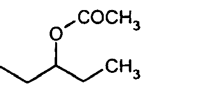 | 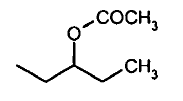 | 0 | Log P = 3,09 |
| 36 | 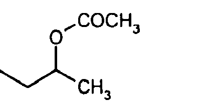 | 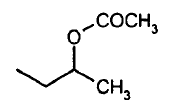 | 0 | Log P = 3,09 |
| 37 | Hydroxymethyl | Hydroxymethyl | 0 | ¹H-NMR (400 MHz, DMSO-d₆): δ = 3,135; 4,789 ppm |
| 38 | Me | Me | 1 | Fp. 205°C |
| 39 | H | Et | 0 | Log P = 2,13 |
| 40 | 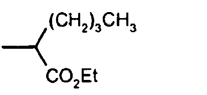 | 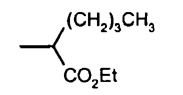 | 0 | Log P = 4,66 |
| 41 | 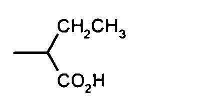 | 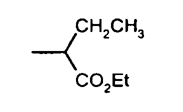 | 0 | Log P = 1,73 |

| | | | | |
|---|---|---|---|---|
| Me = Methyl, Et = Ethyl, nPr = n-Propyl, iPr = Isopropyl, cPr = Cyclopropyl, tBu = tert-Butyl, Bz = Benzyl, Ph = Phenyl | | | | |

### Herstellung von Ausgangstoffen der Formel (IV)

### Herstellung von 4-Oxo-4-{[1-(trifluormethyl)cyclopropyl]amino}butansäure (IV-1)

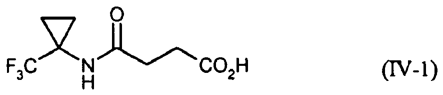

Zu einer Lösung von 496 mg (4,96 mmol) Benrnsteinsäureanhydrid in 10 mL Dioxan gab man unter Eiskühlung (10°C) langsam 800,7 mg (4,96 mmol) 1-(Trifluormethyl)cyclopropanamin und 0,85 mL (4,96 mmol) Diisopropylethylamin zu. Man rührt 20 min. bei Raumtemperatur nach und lässt über Nacht bei dieser Temperatur stehen. Man rührt erneut bei 80°C für 20 min. nach, lässt auf Raumtemperatur abkühlen und engt die Lösung ein. Es wird mehrfach mit Essigsäureethylester und Wasser im Wechsel gewaschen. Die vereinigten organischen Phasen werden getrocknet und eingeengt. Man erhält 815,8 mg (73 % der Theorie) von der gewünschten Verbindung.

Die Bestimmung der in den voranstehenden Tabellen und Herstellungsbeispielen angegebenen Log P-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2,7 mit 0,1 % wässriger Ameisensäure und Acetonitril (enthält 0,1% Ameisensäure) als Eluenten; linearer Gradient von 10% Acetonitril bis 95% Acetonitril

Die Eichung erfolgt mit unverzweigten Alkan-2-onen (mit 3 bis 16 Kohlenstoffatomen), deren logP-Werte bekannt sind (Bestimmung der Log P-Werte anhand der Retentionszeiten durch lineare Interpolation zwischen zwei aufeinanderfolgenden Alkanonen).

### Anwendungsbeispiele

### Beispiel A: Phytophthora-Test (Tomate) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von *Phytophthora infestans* inokuliert. Die Pflanzen werden dann in einer Inkubationskabine bei ca. 20°C und 100% relativer Luftfeuchtigkeit aufgestellt. 3 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen erfindungsgemäß die Verbindungen 1, 2 und 3 bei einer Konzentration an Wirkstoff von 250 ppm einen Wirkungsgrad von 70 % oder mehr.

### Beispiel B: Plasmopara-Test (Rebe) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von *Plasmopara viticola* inokuliert und verbleiben dann 1 Tag in einer Inkubationskabine bei ca. 20°C und 100% relativer Luftfeuchtigkeit. Anschließend werden die Pflanzen 4 Tage im Gewächshaus bei ca. 21°C und ca. 90 % Luftfeuchtigkeit aufgestellt. Die Pflanzen werden dann angefeuchtet und 1 Tag in eine Inkubationskabine gestellt. 6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen erfindungsgemäß die Verbindungen 1, 2 und 3 bei einer Konzentration an Wirkstoff von 250 ppm einen Wirkungsgrad von 70 % oder mehr.

### Beispiel C: Venturia-Test (Apfel) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers *Venturia inaequalis* inokuliert und verbleiben dann 1 Tag bei ca. 20°C und 100 % relativer Luftfeuchtigkeit in einer Inkubationskabine. Die Pflanzen werden dann im Gewächshaus bei ca. 21°C und einer relativen Luftfeuchtigkeit von ca. 90 % aufgestellt. 10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen erfindungsgemäß die Verbindungen 1, 2 und 3 bei einer Konzentration an Wirkstoff von 250 ppm einen Wirkungsgrad von 70 % oder mehr.

### Beispiel D: Alternaria-Test (Tomate) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von *Alternaria solani* inokuliert. Die Pflanzen werden dann in einer Inkubationskabine bei ca. 20°C und 100 % relativer Luftfeuchtigkeit aufgestellt. 3 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen erfindungsgemäß die Verbindungen 1, 2 und 3 bei einer Konzentration an Wirkstoff von 250 ppm einen Wirkungsgrad von 70 % oder mehr.

### Beispiel E: Botrytis-Test (Gurke) / protektiv

| | | |
|---|---|---|
| Lösungsmittel: | 49 | Gewichtsteile N, N-Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von *Botrytis cinerea* inokuliert und stehen dann 48 h bei 100 % relativer Feuchte und 22°C. Anschließend stehen die Pflanzen bei 96 % relativer Luftfeuchtigkeit und einer Temperatur von 14°C. 5-6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen erfindungsgemäß die Verbindungen 1, 2 und 3 bei einer Konzentration an Wirkstotf von 500 ppm einen Wirkungsgrad von 70 % oder mehr.

### Beispiel F: Pyrenophora teres -Test (Gerste) / protektiv

| | | |
|---|---|---|
| Lösungsmittel: | 50 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit einer Sporensuspension von Pyrenophora teres besprüht. Die Pflanzen verbleiben 48 Stunden bei 20°C und 100 % relativer Luftfeuchtigkeit in einer Inkubationskabine. Die Pflanzen werden in einem Gewächshaus bei einer Temperatur von ca. 20°C und einer relativen Luftfeuchtigkeit von ca. 80 % aufgestellt. 8 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigt erfindungsgemäß die Verbindung 1 bei einer Konzentration an Wirkstoff von 1000 ppm einen Wirkungsgrad von 70 % oder mehr.

## Patentansprüche

1. Verwendung von Dithiin-tetracarboximiden der allgemeinen Formel (I) 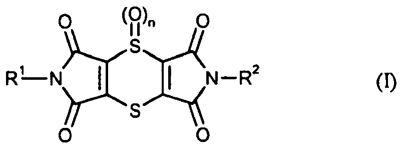 in welcher
R¹ und R² gleich oder verschieden sind und für Wasserstoff, für gegebenenfalls einfach oder mehrfach durch Halogen, -OR³, -COR⁴ substituiertes C₁-C₈-Alkyl, gegebenenfalls ein-fach oder mehrfach durch Halogen, C₁-C₄-Alkyl oder C₁-C₄-Halogenalkyl substituiertes C₃-C₇-Cycloalkyl, jeweils gegebenenfalls einfach oder mehrfach durch Halogen, C₁-C₄-Alkyl, C₁-C₄-Halogenalkyl, -COR⁴ oder Sulfonylamino substituiertes Aryl oder Aryl-(C₁-C₄-alkyl) stehen,
R³ für Wasserstoff, C₁-C₄-Alkyl, C₁-C₄-Alkylcarbonyl oder für gegebenenfalls einfach oder mehrfach durch Halogen, C₁-C₄-Alkyl oder C₁-C₄-Halogenalkyl substituiertes Aryl steht,
R⁴ für Hydroxy, C₁-C₄-Alkyl oder C₁-C₄-Alkoxy steht,
n für 0 oder 1 steht,
zum Bekämpfen phytopathogener Pilze.

2. Verwendung nach Anspruch 1, **dadurch gekennzeichnet, dass**
R¹ und R² gleich oder verschieden sind und für Wasserstoff, für gegebenenfalls einfach oder mehrfach durch Fluor, Chlor, Brom, -OR³, -COR⁴ substituiertes C₁-C₆-Alkyl, gegebenenfalls einfach oder mehrfach durch Chlor, Methyl oder Trifluormethyl substituiertes C₃-C₇-Cycloalkyl, für jeweils gegebenenfalls einfach oder mehrfach durch Fluor, Chlor, Brom, Methyl, Trifluormethyl, -COR⁴, Sulfonylamino substituiertes Phenyl oder Phenyl-(C₁-C₄-alkyl) stehen,
R³ für Wasserstoff, Methyl, Ethyl, Methylcarbonyl, Ethylcarbonyl oder für gegebenenfalls einfach oder mehrfach durch Fluor, Chlor, Methyl, Ethyl, n-Propyl, Isopropyl oder Trifluormethyl substituiertes Phenyl steht,
R⁴ für Hydroxy, Methyl, Ethyl, Methoxy oder Ethoxy steht,
n für 0 oder 1 steht.

3. Verwendung nach Anspruch 1, **dadurch gekennzeichnet, dass**
R¹ und R² gleich oder verschieden sind und für Wasserstoff, für gegebenenfalls einfach oder mehrfach durch Fluor, Chlor, Hydroxy, Methoxy, Ethoxy, Methylcarbonyloxy, Carboxyl substituiertes C₁-C₄Alkyl, gegebenenfalls einfach oder mehrfach durch Chlor, Methyl oder Trifluormethyl substituiertes C₃-C₇-Cycloalkyl, für jeweils gegebenenfalls einfach bis dreifach durch Fluor, Chlor, Brom, Methyl, Trifluormethyl, -COR⁴, Sulfonylamino substituiertes Phenyl, Benzyl, 1-Phenethyl, 2-Phenethyl oder 2-Methyl-2-phenethyl stehen,
R³ für Wasserstoff, Methyl, Methylcarbonyl oder für Phenyl steht,
R⁴ für Hydroxy oder Methoxy steht
n für 0 oder 1 steht.

4. Verwendung gemäß Anspruch 1, **dadurch gekennzeichnet, dass** R1 und R2 gleichzeitig für Methyl stehen.

5. Verfahren zum Bekämpfen phytopathogener Pilze, **dadurch gekennzeichnet, dass** man Dithiin-tetracarboximide der Formel (I) gemäß Anspruch 1 auf die Pilze und/oder deren Lebensraum ausbringt.

6. Dithiin-tetracarboximide der Formel (I-a) 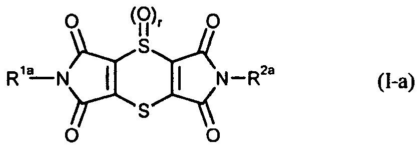 in welcher
R^{1a} und R^{2a} gleich oder verschieden sind und für einfach oder mehrfach durch Fluor, -OR^{3a}, COR^{4a} substituiertes C₁-C₈-Alkyl, gegebenenfalls einfach oder mehrfach durch Halogen, C₁-C₄-Alkyl oder C₁-C₄-Halogenalkyl substituiertes Cyclopropyl, oder für einfach im Alkylteil durch -COR^{4a} substituiertes Aryl-(C₁-C₄-alkyl) stehen,
R^{3a} für C₁-C₄-Alkyl oder für gegebenenfalls einfach oder mehrfach durch Halogen, C₁-C₄-Alkyl oder C₁-C₄-Halogenalkyl substituiertes Aryl steht,
R^{4a} für Hydroxy, C₁-C₄-Alkyl oder C₁-C₄-Alkoxy steht,
r für 0 oder 1 steht,
wobei R^{1a} und R^{2a} nicht gleichzeitig für Acetoxymethyl oder Methoxymethyl stehen.

7. Dithiin-tetracarboximide der Formel (I-a) gemäß Anspruch 6, in welcher
R^{1a} und R^{2a} gleich oder verschieden sind und für einfach oder mehrfach durch Fluor, -OR^{3a}, -COR^{4a} substituiertes C₁-C₆-Alkyl, gegebenenfalls einfach oder mehrfach durch Chlor, Methyl oder Trifluormethyl substituiertes Cyclopropyl, oder für einfach im Alkylteil durch -COR^{4a} substituiertes Phenyl-(C₁-C₄-alkyl) stehen,
R^{3a} für Methyl, Ethyl oder für gegebenenfalls einfach oder mehrfach durch Fluor, Chlor, Methyl, Ethyl, n-Propyl, Isopropyl oder Trifluormethyl substituiertes Phenyl steht,
R^{4a} für Hydroxy, Methyl, Ethyl, Methoxy oder Ethoxy steht,
r für 0 oder 1 steht,
wobei R^{1a} und R^{2a} nicht für Acetoxymethyl stehen.

8. Dithiin-tetracarboximide der Formel (I-a) gemäß Anspruch 6 oder 7, in welcher
R^{1a} und R^{2a} gleich oder verschieden sind und für einfach oder mehrfach durch Fluor, Hydroxy, Methoxy, Ethoxy, Carboxyl substituiertes C₁-C₄-Alkyl, gegebenenfalls einfach oder mehrfach durch Chlor, Methyl oder Trifluormethyl substituiertes Cyclopropyl, oder für einfach im Alkylteil durch -COR^{4a} substituiertes 1-Phenethyl oder 2-Phenethyl stehen,
R^{4a} für Hydroxy oder Methoxy steht,
r für 0 steht,
wobei R^{1a} und R^{2a} nicht für Acetoxymethyl stehen.

9. Verwendung von Dithiin-diisoimiden der Formel (V) 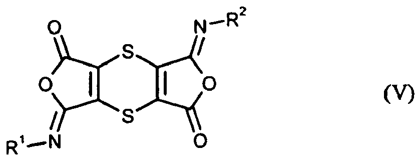 in welcher R¹ und R² die in Anspruch 1 angegebenen Bedeutungen haben,
zum Bekämpfen phytopathogener Pilze.

10. Dithiin-diisoimide der Formel (V-a) 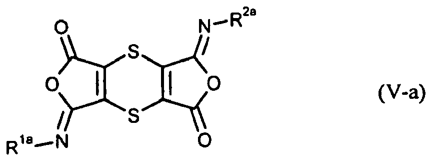 in welcher R^{1a} und R^{2a} die in Anspruch 6 angegebenen Bedeutungen haben.

## Claims

1. Use of dithiine-tetracarboximides of the general formula (I) 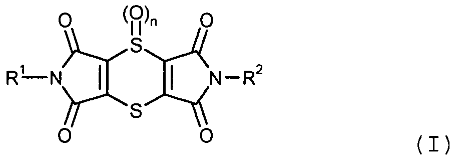 in which
R¹ and R² are identical or different and represent hydrogen, C₁-C₈-alkyl which is optionally monosubstituted or polysubstituted by halogen, -OR³, -COR⁴, or represent C₃-C₇-cycloalkyl which are optionally monosubstituted or polysubstituted by halogen, C₁-C₄-alkyl or C₁-C₄-haloalkyl, or represent aryl or aryl-(C₁-C₄-alkyl), each of which is optionally monosubstituted or polysubstituted by halogen, C₁-C₄-alkyl, C₁-C₄-haloalkyl, -COR⁴ or sulphonylamino,
R³ represents hydrogen, C₁-C₄-alkyl, C₁-C₄-alkylcarbonyl, or represents aryl which is optionally monosubstituted or polysubstituted by halogen, C₁-C₄-alkyl or C₁-C₄-haloalkyl,
R⁴ represents hydroxyl, C₁-C₄-alkyl or C₁-C₄-alkoxy,
n represents 0 or 1
for controlling phytopathogenic fungi.

2. Use according to Claim 1, **characterized in that**
R¹ and R² are identical or different and represent hydrogen, or represent C₁-C₆-alkyl which is optionally monosubstituted or polysubstituted by fluorine, chlorine, bromine, -OR³, -COR⁴, or represent C₃-C₇-cycloalkyl which is optionally monosubstituted or polysubstituted by chlorine, methyl or trifluoromethyl, or represent phenyl or phenyl-(C₁-C₄-alkyl), each of which is optionally monosubstituted or polysubstituted by fluorine, chlorine, bromine, methyl, trifluoromethyl, -COR⁴, sulphonylamino,
R³ represents hydrogen, methyl, ethyl, methylcarbonyl, ethylcarbonyl or represents phenyl which is optionally monosubstituted or polysubstituted by fluorine, chlorine, methyl, ethyl, n-propyl, isopropyl or trifluoromethyl,
R⁴ represents hydroxyl, methyl, ethyl, methoxy or ethoxy,
n represents 0 or 1.

3. Use according to Claim 1, **characterized in that**
R¹ and R² are identical or different and represent hydrogen, or represent C₁-C₄-alkyl which is optionally monosubstituted or polysubstituted by fluorine, chlorine, hydroxyl, methoxy, ethoxy, methylcarbonyloxy, carboxyl, or represent C₃-C₇-cycloalkyl which is optionally monosubstituted or polysubstituted by chlorine, methyl or trifluoromethyl, or represent phenyl, benzyl, 1-phenethyl, 2-phenethyl or 2-methyl-2-phenethyl, each of which is optionally monosubstituted to trisubstituted by fluorine, chlorine, bromine, methyl, trifluoromethyl,-COR⁴, sulphonylamino,
R³ represents hydrogen, methyl, methylcarbonyl or phenyl,
R⁴ represents hydroxyl or methoxy,
n represents 0 or 1.

4. Use according to Claim 1, **characterized in that** R¹ and R² simultaneously represent methyl.

5. Method of controlling phytopathogenic fungi, **characterized in that** dithiine-tetracarboximides of the formula (I) according to Claim 1 are applied to the fungi and/or their environment.

6. Dithiine-tetracarboximides of the formula (I-a) 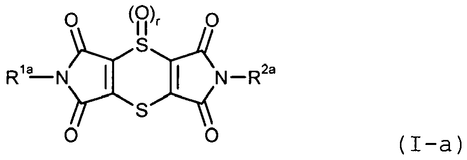 in which
R^{1a} and R^{2a} are identical or different and represent C₁-C₈-alkyl which is monosubstituted or polysubstituted by fluorine, -OR^{3a}, -COR^{4a}, or represent cyclopropyl which is optionally monosubstituted or polysubstituted by halogen, C₁-C₄-alkyl or C₁-C₄-haloalkyl, or represent aryl-(C₁-C₄-alkyl) which is monosubstituted in the alkyl moiety by -COR^{4a},
R^{3a} represents C₁-C₄-alkyl or represents aryl which is optionally monosubstituted or polysubstituted by halogen, C₁-C₄-alkyl or C₁-C₄-haloalkyl,
R^{4a} represents hydroxyl, C₁-C₄-alkyl or C₁-C₄-alkoxy,
r represents 0 or 1,
where R^{1a} and R^{2a} do not simultaneously represent acetoxymethyl or methoxymethyl.

7. Dithiine-tetracarboximides of the formula (I-a) according to Claim 6, in which
R^{1a} and R^{2a} are identical or different and represent C₁-C₆-alkyl which is monosubstituted or polysubstituted by fluorine, -OR^{3a}, -COR^{4a} or represent cyclopropyl which is optionally monosubstituted or polysubstituted by chlorine, methyl or trifluoromethyl, or represent phenyl-(C₁-C₄-alkyl) which is monosubstituted in the alkyl moiety by -COR^{4a},
R^{3a} represents methyl, ethyl, or represents phenyl which is optionally monosubstituted or polysubstituted by fluorine, chlorine, methyl, ethyl, n-propyl, isopropyl or trifluoromethyl,
R^{4a} represents hydroxyl, methyl, ethyl, methoxy or ethoxy,
r represents 0 or 1,
where R^{1a} and R^{2a} do not represent acetoxymethyl.

8. Dithiine-tetracarboximides of the formula (I-a)
according to Claim 6 or 7, in which
R^{1a} and R^{2a} are identical or different and represent C₁-C₄-alkyl which is monosubstituted or polysubstituted by fluorine, hydroxyl, methoxy, ethoxy, carboxyl, or represent cyclopropyl which is optionally monosubstituted or polysubstituted by chlorine, methyl or trifluoromethyl, or represent 1-phenethyl or 2-phenethyl, each of which is monosubstituted in the alkyl moiety by -COR^{4a},
R^{4a} represents hydroxyl or methoxy,
r represents 0,
where R^{1a} and R^{2a} do not represent acetoxymethyl.

9. Use of dithiine-diisoimides of the formula (V) 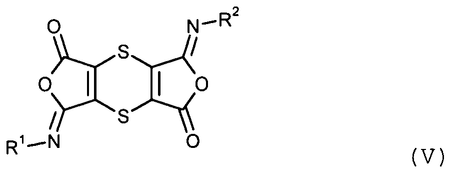 in which R¹ and R² have the meanings given in Claim 1
for controlling phytopathogenic fungi.

10. Dithiine-diisoimides of the formula (V-a) 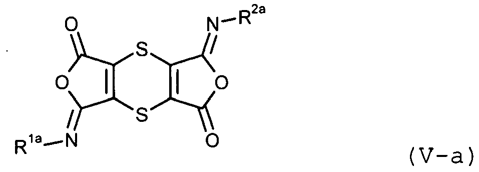 in which R^{1a} and R^{2a} have the meanings given in Claim 6.

## Revendications

1. Utilisation de dithiine-tétracarboximides de formule générale (I) 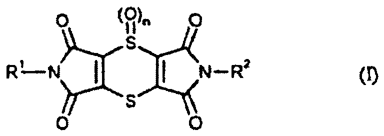 dans laquelle
R¹ et R² sont identiques ou différents et représentent l'hydrogène, un alkyle en C₁-C₈ éventuellement substitué une ou plusieurs fois par halogène, -OR³, -COR⁴, un cycloalkyle en C₃-C₇ éventuellement substitué une ou plusieurs fois par halogène, alkyle en C₁-C₄ ou halogénoalkyle en C₁-C₄, un aryle ou aryl-(alkyle en C₁-C₄) chacun éventuellement substitué une ou plusieurs fois par halogène, alkyle en C₁-C₄, halogénoalkyle en C₁-C₄, -COR⁴ ou sulfonylamino,
R³ représente l'hydrogène, un alkyle en C₁-C₄, un alkylcarbonyle en C₁-C₄ ou un aryle éventuellement substitué une ou plusieurs fois par halogène, alkyle en C₁-C₄ ou halogénoalkyle en C₁-C₄,
R⁴ représente hydroxy, un alkyle en C₁-C₄ ou un alcoxy en C₁-C₄,
n représente 0 ou 1,
pour lutter contre les champignons phytopathogènes.

2. Utilisation selon la revendication 1, **caractérisée en ce que**
R¹ et R² sont identiques ou différents et représentent l'hydrogène, un alkyle en C₁-C₆ éventuellement substitué une ou plusieurs fois par fluor, chlore, brome, -OR³, - COR⁴, un cycloalkyle en C₃-C₇ éventuellement substitué une ou plusieurs fois par chlore, méthyle ou trifluorométhyle, un phényle ou phényl-(alkyle en C₁-C₄) chacun éventuellement substitué une ou plusieurs fois par fluor, chlore, brome, méthyle, trifluorométhyle, - COR⁴, sulfonylamino,
R³ représente l'hydrogène, méthyle, éthyle, méthylcarbonyle, éthylcarbonyle ou un phényle éventuellement substitué une ou plusieurs fois par fluor, chlore, méthyle, éthyle, n-propyle, isopropyle ou trifluorométhyle,
R⁴ représente hydroxy, méthyle, éthyle, méthoxy ou éthoxy,
n représente 0 ou 1.

3. Utilisation selon la revendication 1, **caractérisée en ce que**
R¹ et R² sont identiques ou différents et représentent l'hydrogène, un alkyle en C₁-C₄ éventuellement substitué une ou plusieurs fois par fluor, chlore, hydroxy, méthoxy, éthoxy, méthylcarbonyloxy, carboxyle, un cycloalkyle en C₃-C₇ éventuellement substitué une ou plusieurs fois par chlore, méthyle ou trifluorométhyle, un phényle, benzyle, 1-phénéthyle, 2-phénéthyle ou 2-méthyl-2-phénéthyle chacun éventuellement substitué une ou plusieurs fois par fluor, chlore, brome, méthyle, trifluorométhyle, -COR⁴, sulfonylamino,
R³ représente l'hydrogène, méthyle, méthylcarbonyle ou phényle,
R⁴ représente hydroxy ou méthoxy,
n représente 0 ou 1.

4. Utilisation selon la revendication 1, **caractérisée en ce que** R¹ et R² représentent simultanément méthyle.

5. Procédé de lutte contre les champignons phytopathogènes, **caractérisé en ce que** des dithiine-tétracarboximides de formule (I) selon la revendication 1 sont appliqués sur les champignons et/ou leur habitat.

6. Dithiine-tétracarboximides de formule (I-a) 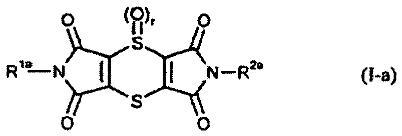 dans laquelle
R^{1a} et R^{2a} sont identiques ou différents et représentent un alkyle en C₁-C₈ substitué une ou plusieurs fois par fluor, -OR^{3a}, -COR^{4a}, un cyclopropyle éventuellement substitué une ou plusieurs fois par halogène, alkyle en C₁-C₄ ou halogénoalkyle en C₁-C₄, ou un aryl-(alkyle en C₁-C₄) substitué une fois dans la partie alkyle par - COR^{4a},
R^{3a} représente un alkyle en C₁-C₄ ou un aryle éventuellement substitué une ou plusieurs fois par halogène, alkyle en C₁-C₄ ou halogénoalkyle en C₁-C₄,
R^{4a} représente hydroxy, alkyle en C₁-C₄ ou alcoxy en C₁-C₄,
r représente 0 ou 1,
R^{1a} et R^{2a} ne représentant pas simultanément acétoxyméthyle ou méthoxyméthyle.

7. Dithiine-tétracarboximides de formule (I-a) selon la revendication 6, dans lesquelles
R^{1a} et R^{2a} sont identiques ou différents et représentent un alkyle en C₁-C₆ substitué une ou plusieurs fois par fluor, -OR^{3a}, -COR^{4a}, un cyclopropyle éventuellement substitué une ou plusieurs fois par chlore, méthyle ou trifluorométhyle, ou un phényl-(alkyle en C₁-C₄) substitué une fois dans la partie alkyle par -COR^{4a}
R^{3a} représente méthyle, éthyle ou un phényle éventuellement substitué une ou plusieurs fois par fluor, chlore, méthyle, éthyle, n-propyle, isopropyle ou trifluorométhyle,
R^{4a} représente hydroxy, méthyle, éthyle, méthoxy ou éthoxy,
r représente 0 ou 1,
R^{1a} et R^{2a} ne représentant pas acétoxyméthyle.

8. Dithiine-tétracarboximides de formule (I-a) selon la revendication 6 ou 7, dans lesquelles R^{1a} et R^{2a} sont identiques ou différents et représentent un alkyle en C₁-C₄ substitué une ou plusieurs fois par fluor, hydroxy, méthoxy, éthoxy, carboxyle, un cyclopropyle éventuellement substitué une ou plusieurs fois par chlore, méthyle ou trifluorométhyle, ou un 1-phénéthyle ou 2-phénéthyle substitué une fois dans la partie alkyle par -COR^{4a},
R^{4a} représente hydroxy ou méthoxy,
r représente 0,
R^{1a} et R^{2a} ne représentant pas acétoxyméthyle.

9. Utilisation de dithiine-diisoimides de formule (V) 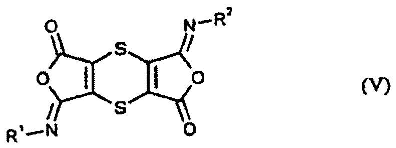 dans laquelle R¹ et R² ont les significations données dans la revendication 1,
pour lutter contre les champignons phytopathogènes.

10. Dithiine-diisoimides de formule (V-a) 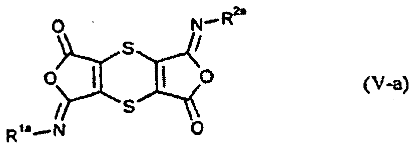 dans laquelle R^{1a} et R^{2a} ont les significations données dans la revendication 6.
